# EUROPEAN PATENT APPLICATION

(11) **EP 1 433 750 A1**
(43) Date of publication of application: **30.06.2004**
(21) Application number: 03029601.6
(22) Date of filing: 22.12.2003
(51) Int. Cl.: C01F 17/00, C09K 9/00, C09K 11/82

(54) **Ornamental material performing particular light emission or particular color emission**

(30) Priority: 26.12.2002 JP 2002376386; 26.06.2003 JP 2003182665
(71) Applicant: Nec Tokin Corporation, Sendai-shi, Miyagi (JP)
(72) Inventor: Itagaki, Hitoshi, Sendai-shi Miyagi (JP); Machida, Hiroshi, Sendai-shi Miyagi (JP)
(74) Representative: Hofer, Dorothea, Dipl.-Phys.

(57) **Abstract**

As an ornamental material, use is made of a single crystal represented by a chemical formula RVO₄, where R is at least one rare earth element selected from Sc, Y, La, Ce, Pr, Nd, Pm, Sm, Eu, Gd, Tb, Dy, Ho, Er, Tm, Yb, and Lu.

## Description

This application claims priority to prior Japanese applications JP 2002-376386 and 2003-182665, the disclosures of which are incorporated herein by reference.

### Background of the Invention:

This invention relates to an ornamental material which performs light emission or color emission under the light. It is noted here that, in the present specification, the "ornamental material" includes a single crystal material and various single crystal products obtained from the single crystal material, such as a single crystal block, fine crystals, and powder particles as well as a combination of the single crystal product and any other raw material. For example, the single crystal material or the various single crystal product may be dispersed in other raw material, arranged at a part of an ornamental product, or adhered to a fiber-like product.

Precious metals or precious stones (jewelry) are often used as ornamental materials for accessories, such as necklaces and earrings, various artistic shaped articles (so-called objets d'art in French), and interior goods. Generally, however, such ornamental materials do not emit light in the darkness. Further, such ornamental materials do not perform light emission or color emission even under a light source emitting electromagnetic waves, such as the ultraviolet light (for example, the black light) or the infrared light, different in wavelength from the typical visible light. As known in the art, the black light is near-ultraviolet radiation or special-wavelength ultraviolet light.

In order to achieve light emission or color emission under the ultraviolet light or the infrared light other than the visible light, it is proposed to add a very small amount of additional elements to crystals of various ornamental materials, typically, diamonds. At present, however, any technique practically usable is not yet obtained. On the other hand, a material which emits light when excited by the ultraviolet light is disclosed, for example, in Japanese Patent Application Publication (JP-A) No. 2001-107045 (corresp. to USP 6,590,333).

The above-mentioned publication discloses the phosphor represented by a chemical formula Ln(P_{c}V_{1-c})O₄•(MO₂)_{d}. Herein, Ln is at least one element selected from Sc, Y, La, Ce, Pr, Nd, Pm, Sm, Eu, Gd, Tb, Dy, Ho, Er, Tm, Yb, Lu, B, Al, Ga, In, and TI while M is at least one element selected from those elements belonging to Group IV.

The material disclosed in the above-mentioned publication is excited by the ultraviolet light and changed in composition to emit fluorescence in various colors. However, the above-mentioned material is obtained by baking or firing a powder material having a predetermined composition and is therefore obtained in a powdery shape. Thus, it is difficult to obtain this material as a massive crystal which is easily processed into a desired shape.

Besides the above-mentioned publication, various techniques for obtaining a phosphor are known. In any of these techniques, however, the massive crystal is not obtained. Therefore, the use of the phosphor is restricted to a phosphor thin film of a plasma display panel or an ornament for a glass container or vessel.

### Summary of the Invention:

It is therefore an object of the present invention to provide an ornamental material which can easily be processed into various shapes.

It is another object of the present invention to provide an ornamental material of the type described, which is capable of performing light emission or color emission in different colors under different types of lights, such as the visible light, the ultraviolet light, and the infrared light.

It is a more specific object of the present invention to provide an ornamental material which is capable of performing, when irradiated by the ultraviolet light or the infrared light, light emission or color emission in a color different from that exhibited under the visible light and which has a dimension adapted to be processed into a single crystal block, single crystal pieces, fine crystals, and powder particles for the purpose of use as accessories, nail art materials, manicure, beads, resin molds, glass products, cosmetics, paints, fiber, woven fabric, nonwoven fabric, and so on.

In order to achieve the above-mentioned object, the present inventors focused upon a single crystal made from vanadium oxide and oxide of a rare earth element as raw materials and this invention has been made.

According to a first aspect of the present invention, there is provided an ornamental material comprising a single crystal represented by a chemical formula RVO₄, where R is at least one rare earth element selected from Sc, Y, La, Ce, Pr, Nd, Pm, Sm, Eu, Gd, Tb, Dy, Ho, Er, Tm, Yb, and Lu.

According to a second aspect of the present invention, there is provided an ornamental material having powder particles obtained by processing a single crystal represented by a chemical formula RVO₄, where R is at least one rare earth element selected from Sc, Y, La, Ce, Pr, Nd, Pm, Sm, Eu, Gd, Tb, Dy, Ho, Er, Tm, Yb, and Lu.

According to a third aspect of the present invention, there is provided an ornamental material having fine crystals obtained by processing a single crystal represented by a chemical formula RVO₄, where R is at least one rare earth element selected from Sc, Y, La, Ce, Pr, Nd, Pm, Sm, Eu, Gd, Tb, Dy, Ho, Er, Tm, Yb, and Lu.

According to a fourth aspect of the present invention, there is provided an ornamental material exhibiting different colors under the ultraviolet light, the infrared light, and the visible light, respectively.

According to a fifth aspect of the present invention, there is provided an ornamental material exhibiting, when irradiated by one of the ultraviolet light and the infrared light, a color different from that exhibited under the visible light.

According to a sixth aspect of the present invention, there is provided an ornamental material comprising 40.0-60.0 mol% vanadium oxide and 40.0-60.0 mol% rare earth element or elements. The ornamental material can be obtained by a typical single crystal growth method. The single crystal growth method generally comprises the steps of mixing powder of vanadium oxide and powder of the rare earth element or elements to prepare a mixture, baking or firing the mixture to produce a baked product, melting the baked product into a melt, and solidifying the melt.

For the growth of the single crystal used in the ornamental material of this invention, use is made of an air atmosphere, an oxygen atmosphere, a mixed atmosphere of an inactive gas and oxygen as a growth atmosphere. The characteristics of the ornamental material of this invention mainly depend upon the composition of the single crystal while the color exhibited upon light emission or color emission is different depending upon the growth atmosphere. Therefore, it is preferable to appropriately control the growth atmosphere depending upon the kind of the raw materials. The single crystal obtained by the growth method is subjected to heat treatment so that the single crystallinity, in particular, the refractive index uniformity is improved and the ornamental material having more excellent characteristics can be obtained. Therefore, it is also preferable to consider and determine an appropriate condition for the heat treatment after the growth of the crystal.

The ornamental material comprising the single crystal subjected to the above-mentioned treatment is processed into any desired shape, such as a single crystal block, single crystal pieces, fine crystals, powder particles, and so on. In this manner, it is possible to produce accessories, nail art products, manicure, glass products, resin molds, cosmetic products, woven fabric, nonwoven fabric, fiber products, and so on which exhibit color emission or color change unique and unknown in the art.

### Description of the Preferred Embodiments:

Now, description will be made of preferred embodiments of the present invention in conjunction with specific examples.

In the specific examples of this invention which will hereinafter be described, V₂O₅ (vanadium oxide) and at least one oxide of rare earth element as raw materials were weighed so that each of the amount of vanadium oxide and the total amount of at least one oxide of rare earth element fell within a range of 40.0-60.0 mol%. These raw materials were wet-mixed to prepare a powder material. Next, the powder material was baked or fired. By the use of the powder material baked or fired, a rod-like material was produced and melted into a melted material. From the melted material, a single crystal was grown as an ornamental material of this invention.

### First Example

In the first example of this invention, V₂O₅, Er₂O₃ (erbium oxide), and Gd₂O₃ (gadolinium oxide) were used as raw materials. At first, the raw materials were weighed so that V₂O₅, Er₂O₃, Gd₂O₃ fell within ranges of 40.0-60.0 mol%, 0.3-7.0 mol%, and 38.0-54.7 mol%, respectively, to prepare a powder material. By the use of the powder material, a single crystal was grown in the above-mentioned manner. The single crystal as the ornamental material thus obtained exhibited pink color when irradiated by the fluorescent light as the visible light and emitted white light and yellowish green light when irradiated by the sunlight as the infrared light and when irradiated by the black light as the ultraviolet light, respectively.

Preferably, V₂O₅, Er₂O₃, Gd₂O₃ fell within ranges 47.0-53.0 mol%, 0.5-4.0 mol%, and 43.0-53.0 mol%, respectively. In these ranges, the crystallinity is more excellent and the transparency and the coloration are improved.

### Second Example

In the second example of this invention, V₂O₅, Y₂O₃ (yttrium oxide), and Gd₂O₃ were used as raw materials. At first, the raw materials were weighed so that V₂O₅, Y₂O₃, Gd₂O₃ fell within ranges of 45.0-55.5 mol%, 0-55.0 mol% (0 exclusive), and 0-55.0 mol% (0 exclusive), respectively, to prepare a powder material. By the use of the powder material, a single crystal was grown in the above-mentioned manner. The single crystal as the ornamental material thus obtained was colorless under the fluorescent light and the sunlight but emitted bluish white light under the black light.

Preferably, V₂O₅, Y₂O₃, Gd₂O₃ fell within ranges of 47.0-51.0 mol%, 0.3-20.0 mol%, and 29.0-53.0 mol%, respectively. In these ranges, the crystallinity is more excellent and the transparency and the coloration are improved.

### Third Example

In the third example, V₂O₅, Eu₂O₃ (europium oxide), Nd₂O₃ (neodymium oxide), and Gd₂O₃ were used as raw materials. At first, the raw materials were weighed so that V₂O₅, Eu₂O₃, Nd₂O₃, and Gd₂O₃ fell within ranges of 45.0-55.5 mol%, 0.1-8.0 mol%, 0.1-10.0 mol%, and 27.0-54.8 mol%, respectively, to prepare a powder material. By the use of the powder material, a single crystal was grown in the above-mentioned manner. The single crystal as the ornamental material thus obtained exhibited blue color under the fluorescent light. Under the black light and the sunlight, the single crystal emitted reddish pink light and violet light, respectively. Thus, the same single crystal as the ornamental material exhibited different colors under the visible light, the ultraviolet light, and the infrared light.

Preferably, V₂O₅, Eu₂O₃, Nd₂O₃, and Gd₂O₃ fell within ranges of 47.0-51.0 mol%, 0.5-4.0 mol%, 0.5-5.0 mol%, and 40.0-52.0 mol%, respectively. In these ranges, the crystallinity is more excellent and the transparency and the coloration are improved.

### Fourth Example

By the use of raw materials similar to those of the third example, a single crystal was grown in the above-mentioned manner. The single crystal was cut or pulverized into single crystal pieces having the dimension of 1mm square to 0.3mm square and the thickness of about 0.2mm. The single crystal pieces were applied and adhered to a nail together with a commercially available nail art material. Thus, the single crystal pieces as the ornamental material were used as a nail art material.

It has been confirmed that the above-mentioned ornamental material exhibited different colors under the fluorescent light, the black light, and the sunlight and, therefore, can be used as a distinct and unique nail art material.

### Fifth Example

By the use of raw materials similar to those of the third example, a single crystal was grown in the above-mentioned manner. The single crystal was processed into fine crystals or powder particles to be used as a particular one of glass materials. By the use of the particular glass material, a glass product was produced. The fine crystals or the powder particles as the ornamental material were distributed and dispersed in the glass product as single crystals and exhibited color change in response to different kinds of lights. Thus, unlike existing glass products, the glass product in this example exhibits different colors under the fluorescent light, the sunlight, and the black light, respectively. Since the single crystals have a melting point higher than a glass melting temperature and since the particular glass material does not have reactivity with the other conventional glass material, the characteristics of the particular glass material were maintained.

By the use of the glass product, handicraft beads were prepared. It has been confirmed that the similar visual effect could be obtained.

### Sixth Example

By the use of raw materials similar to those of the third example, a single crystal was grown in the above-mentioned manner. The single crystal was processed into fine crystals or powder particles to be used as a particular one of resin mold coloring materials. By the use of the particular resin mold coloring material, a resin mold was produced.

The fine crystals or the powder particles as the ornamental material were distributed and dispersed as single crystals within the resin mold and exhibited color change in response to irradiation by different kinds of lights. Unlike existing resin mold products, the resin mold product in this example exhibits different colors under the fluorescent light, the sunlight, and the black light, respectively. Since the single crystals have a melting point higher than a resin melting temperature and since the particular resin mold coloring material does not have reactivity with the resin, the characteristics of the particular resin mold coloring material were maintained.

By the use of the resin mold product, handicraft resin beads were prepared. It has been confirmed that the similar visual effect could be obtained.

### Seventh Example

By the use of raw materials similar to those of the third example, a single crystal was grown in the above-mentioned manner. The single crystal was processed into a single crystal plate of a flat shape. Alternatively, the single crystal was processed into crystal pieces or fine crystals to be dispersed in a glass. By the use of the single crystal plate or the glass with the crystal pieces or the fine crystals dispersed therein, a sensor for the infrared light and the ultraviolet light was produced.

By the use of the single crystal as the ornamental material exhibiting blue color under the visible light and instantaneously changing its color into red color or violet color in response to the infrared light or the ultraviolet light, it is possible to instantaneously detect the kind and the strength of an irradiated light with reference to the change in color which is visually observed.

### Eighth Example

By the use of raw materials similar to those of the third example, a single crystal was grown in the above-mentioned manner. The single crystal was processed into powder particles. The powder particles were mixed with other raw materials and subjected to a typical process to prepare a cosmetic material as the ornamental material. By the use of the cosmetic material, a cosmetic product different in color under different kinds of irradiated lights was produced.

The cosmetic product, such as a foundation, an eye shadow, a face color, a mascara, and an eyebrow produced by the use of the above-mentioned cosmetic material was applied to a human skin and color emission under the black light, the sunlight, and the fluorescent light was confirmed. Under these different light sources, different colors were exhibited to achieve cosmetic effects depending upon the situations.

### Ninth Example

By the use of raw materials similar to those of the third example, a single crystal was grown in the above-mentioned manner. The single crystal was processed into powder particles and mixed with other raw materials and subjected to a typical process to prepare a manicure material as the ornamental material. By the use of the manicure material, a manicure different in color under different kinds of irradiated lights was produced.

The manicure thus obtained was applied to a nail and color emission under the black light, the sunlight, and the fluorescent light was confirmed. Under the different kinds of light sources, different colors were exhibited to achieve cosmetic effects depending upon the situations.

### Tenth Example

By the use of raw materials similar to those of the third example, a single crystal was grown in the above-mentioned manner. The single crystal was processed into powder particles as the ornamental material and attached to a fiber product. The fiber product was formed into woven fabric or nonwoven fabric.

It has been confirmed that such fabric products exhibited different colors under the visible light, the ultraviolet light, and the infrared light. For example, it is possible to produce clothing which is colorless and transparent under the visible light and the infrared light and which produces an emerging pattern when irradiated by the ultraviolet light, and a clothing exhibiting blue color under the visible light, violet color under the infrared light, and pink to red under the ultraviolet light. Furthermore, it is possible to produce clothing comprising a plurality of kinds of the omamental materials and exhibiting quite different colors/pattems in response to different kinds of irradiated lights. Specifically, the colors/patterns are changed among blue, green, pink, and red as basic colors when irradiated by the ultraviolet light.

As thus far been described in conjunction with the several specific examples of the ornamental material according to this invention, the ornamental material is not restricted to the above-mentioned examples but may be widely applicable to any other products having ornamental effects, such as daily goods, clothes, bags, clothing including small articles, paper products including wall papers and wrapping papers, building materials including glass plates and resin plates, and so on.

Generally, the visible light has a wavelength range from 360-400nm to 760-830nm. The ultraviolet light has a wavelength range from 360-400nm (as a short-wavelength limit of the visible light) to about 1 nm. The infrared light has a wavelength range from 760-830 nm (as a long-wavelength limit of the visible light) to about 1 mm. As an infrared source, the sun is known. The sun's spectrum encompasses the visible spectrum and spans over the infrared light, the ultraviolet light, the radio wave, and the X-ray. The fluorescent light is used as a light source for obtaining a nearly white light.

As described above, the ornamental material according to this invention exhibits clear color under the fluorescent light, like the existing ornamental materials, and performs light emission or color emission even under the black light or the sunlight, unlike the existing ornamental materials. Furthermore, the ornamental material according to this invention is adapted to exhibit different colors under the visible light (for example, the fluorescent light), the ultraviolet light (for example, the black light), and the infrared light (for example, the sunlight).

From the above-mentioned characteristics, the ornamental material according to this invention has a very wide range of applications, for example, accessories including necklaces, earrings, and bracelets, daily goods including clocks and watches, key holders, mobile apparatuses, miscellaneous goods and small articles, interior goods and artistic shaped articles (objets d'art in French) including furniture and large clocks, stationery, table ware, fiber products including papers and cloths. The ornamental material may be used in various manners, for example, embedded into, dispersed in, and adhered to those goods and products. Thus, the ornamental material of this invention is suitable for use as novel ornamental and decorative products.

Furthermore, the ornamental material of this invention is widely applicable to a product having a sensor function of detecting presence/absence of the ultraviolet light and the infrared light as well as the strength thereof.

## Claims

1. An ornamental material comprising a single crystal represented by a chemical formula RVO₄, where R is at least one rare earth element selected from Sc, Y, La, Ce, Pr, Nd, Pm, Sm, Eu, Gd, Tb, Dy, Ho, Er, Tm, Yb, and Lu.

2. An ornamental material according to claim 1, wherein the single crystal is used in the form of a block.

3. An ornamental material having powder particles-obtained by processing a single crystal represented by a chemical formula RVO₄, where R is at least one rare earth element selected from Sc, Y, La, Ce, Pr, Nd, Pm, Sm, Eu, Gd, Tb, Dy, Ho, Er, Tm, Yb, and Lu.

4. An ornamental material having fine crystals obtained by processing a single crystal represented by a chemical formula RVO₄, where R is at least one rare earth element selected from Sc, Y, La, Ce, Pr, Nd, Pm, Sm, Eu, Gd, Tb, Dy, Ho, Er, Tm, Yb, and Lu.

5. An ornamental material according to any one of claims 1 through 4, wherein the ornamental material performs, when excited by one of the infrared light and the ultraviolet light, one of light emission and color emission in a color different from that exhibited under the visible light.

6. An ornamental material according to any one of claims 1 through 4, wherein the ornamental material exhibits different colors under the ultraviolet light, the infrared light, and the visible light, respectively.

7. An ornamental material according to any one of claims 1 through 4, wherein the ornamental material exhibits, when irradiated by one of the ultraviolet light and the infrared light, a color different from that exhibited under the visible light.

8. An ornamental material according to any one of claims 1 through 4, wherein the ornamental material instantaneously causes color change when irradiated light directed to the ornamental material is switched among the ultraviolet light, the infrared light, and the visible light.

9. An ornamental material exhibiting different colors under the ultraviolet light, the infrared light, and the visible light, respectively.

10. An ornamental material exhibiting, when irradiated by one of the ultraviolet light and the infrared light, a color different from that exhibited under the visible light.

11. An ornamental material comprising 40.0-60.0 mol% vanadium oxide and 40.0-60.0 mol% rare earth element.

12. An ornamental material according to any one of claims 1 through 11, wherein the ornamental material is used as one of synthetic jewelry, a nail art material, manicure, a pigment, a cosmetic product, a coating material, beads, a glass product, a resin mold, woven fabric, nonwoven fabric, and a fiber product.

13. An ornamental material according to any one of claims 1 through 12, wherein the ornamental material serves as one of an ultraviolet sensor and an infrared sensor.
